# DEMANDE DE BREVET EUROPEEN

(11) **EP 3 062 251 A1**
(43) Date de publication de la demande: **31.08.2016**
(21) Numéro de dépôt: 16156915.7
(22) Date de dépôt: 23.02.2016
(51) Int. Cl.: G06F 19/00, G01N 35/00

(54) **SYSTEME POUR L'ANALYSE D'ECHANTILLONS POUVANT ÊTRE UTILISE PAR DES LABORATOIRES DE BIOLOGIE**

(30) Priorité: 25.02.2015 FR 1551612
(71) Demandeur: Biomnis, Lyon (FR)
(72) Inventeur: BESSON, Michel L., 69003 Lyon (FR); PAYEUR, SergeE G., Vallangoujard (FR)
(74) Mandataire: Delumeau, François Guy

(57) **Abrégé**

Ce système pour l'analyse d'échantillons comporte un équipement centralisé **(10)** relié à un système d'information **(75)** d'un laboratoire de biologie sous-traitant **(5)** et au moins un équipement distant **(50)** installé dans un laboratoire de biologie commanditaire **(6).**

L'équipement du laboratoire commanditaire s'interface avec un concentrateur **(706)** d'automates d'analyses d'échantillons **(80)** de ce laboratoire et envoie, via un réseau de communication **(1),** une demande d'analyse au laboratoire sous-traitant.

La demande d'analyse **(DA)** est formatée selon un format normalisé de présentation de données biologiques **(PRES).**

Le résultat de l'analyse est reçu dans le laboratoire commanditaire via le concentrateur **(706).**

## Description

### Arrière-plan de l'invention

L'invention se rapporte au domaine général du traitement des prélèvements d'échantillons au sein d'un laboratoire de biologie.

Dans l'état actuel des choses, il est fréquent qu'un laboratoire dit « commanditaire » soit amené à sous-traiter des analyses auprès d'un laboratoire « sous-traitant » par exemple lorsqu'il ne dispose pas localement d'un automate apte à réaliser cette analyse, ou d'un agrément l'autorisant à réaliser cette analyse.

Cette opération consiste, pour le laboratoire commanditaire, à envoyer au laboratoire sous-traitant, en parallèle d'un ou plusieurs tubes comportant les échantillons à analyser, une demande informatique précisant le contexte et les analyses devant être effectuées sur ces échantillons. De façon classique, cette demande informatique se matérialise par l'envoi sécurisé d'un fichier, par exemple par email. Ces traitements par le laboratoire commanditaire et par le laboratoire sous-traitant dépendent fortement des systèmes d'informations mis en place dans ces laboratoires, ceux-ci n'étant pas homogènes.

Un des buts de l'invention est d'apporter des améliorations à cette situation.

### Objet et résumé de l'invention

Plus précisément l'invention concerne un système pour l'analyse d'échantillons, ce système comportant :
- un équipement centralisé relié au système d'information d'un laboratoire de biologie sous-traitant opérant au moins un automate d'analyses d'échantillons; et
- au moins un équipement distant destiné à être installé dans un laboratoire de biologie commanditaire, l'équipement distant étant relié à l'équipement centralisé par un réseau de communication.

Conformément à l'invention, cet équipement distant comporte :
- un premier module de communication connecté à un concentrateur d'automates d'analyses d'échantillons et apte à recevoir du système d'information du laboratoire commanditaire, via le concentrateur, et conformément à un protocole standardisé interprétable par le concentrateur, une demande d'analyse pouvant être réalisée par un automate du laboratoire sous-traitant ; et
- un deuxième module de communication apte à envoyer la demande d'analyse à un module de communication de l'équipement centralisé et à recevoir en réponse un résultat d'analyse ;
- le premier module de communication étant apte à envoyer le résultat d'analyse au système d'information du laboratoire commanditaire via le concentrateur précité selon le protocole standardisé.

Conformément à l'invention, le système comporte un module de conversion apte à :
- convertir la demande d'analyse selon un format normalisé de présentation de données biologiques pour traitement par un système d'information du laboratoire de biologie sous-traitant ; et à :
- convertir ledit résultat d'analyse conformément au protocole standardisé pour que ce résultat puisse être communiqué au système d'information du laboratoire commanditaire via le concentrateur de ce laboratoire.

Ainsi, et de façon très avantageuse, l'invention propose une solution permettant à un laboratoire de biologie de communiquer avec un laboratoire de biologie distant, en utilisant un équipement (« équipement distant au sens de l'invention ») s'interfaçant directement avec le concentrateur d'automates de ce laboratoire, et en utilisant un protocole standardisé interprétable par le concentrateur et une norme de présentation conventionnellement utilisés dans les laboratoires de biologie, en local.

La solution conforme à l'invention est ainsi avantageusement indépendante du système d'informations du laboratoire commanditaire.

Le protocole standardisé interprétable par le concentrateur d'automates peut par exemple être le protocole ASTM ou le protocole HL7 (en anglais « Health Level Seven ») version v2.x ou supérieure ou le protocole HL7 CDA.

Le format normalisé de présentation de données biologiques est par exemple le format HPRIM Santé version 2.x ou supérieure.

Pour plus de renseignements sur le protocole ASTM, l'homme du métier peut se reporter au document « Standard Specification for Transferring Clinical Observations Between Independant Computer Systems » portant la désignation E1238-97 et disponible auprès de ASTM International.

En ce qui concerne les protocoles HL7 V2.X et HL7 CDA, l'homme du métier peut se reporter aux documents publiés aux adresses [1] et [2] ci-dessous :
[1]:http://www.hl7.org/implement/standards/product brief.cfm?product id= 185
[2]:http://www.hl7.org/implement/standards/product_brief.cfm?product_id= 7.

En ce qui concerne, le format HPRIM Santé, version 2.4, l'homme du métier peut se reporter au document « HPRIM Santé, version 2.4 de février 2013 - Application et évolution de la Norme ASTM (E1238 - Draft Révision 4.2) pour l'échange des données formatées entre Laboratoires d'Analyses Médicales, Etablissements Cliniques et Hospitaliers, Etablissements de Transfusion Sanguine, et Cabinets de Radiologie ».

L'invention permet en outre aux utilisateurs du laboratoire commanditaire de traiter les demandes d'analyses réalisées en local et celles sous-traitées au laboratoire sous-traitant de façon strictement identiques.

Autrement dit, l'équipement du laboratoire commanditaire conforme à l'invention utilisé pour envoyer/recevoir des demandes/résultats d'analyse au/du laboratoire d'analyses sous-traitant est vu comme un automate déporté du laboratoire commanditaire rattaché au concentrateur.

Dans un mode particulier de réalisation, le deuxième module de communication du laboratoire commanditaire obtient automatiquement les résultats d'analyse du laboratoire sous-traitant dès que ceux-ci sont disponibles.

Cette caractéristique permet avantageusement au laboratoire commanditaire d'obtenir, pour une demande complexe, des résultats partiels dès que ceux-ci sont disponibles.

Dans un mode particulier de l'invention, le module de communication de l'équipement centralisé et le deuxième module de communication de l'équipement distant encapsulent respectivement la demande d'analyse et le résultat d'analyse dans un Service Web, par exemple conforme au protocole JSON et/ou REST.

Ainsi, l'invention permet, dans ce mode de réalisation, une communication entre les équipements centralisé et distant via le réseau Internet, de façon simple, via le protocole HTTP, éventuellement entre des équipements de fabricants différents, l'équipement distant étant alors constitué d'un serveur Web apte à dialoguer avec le système d'information central du laboratoire sous-traitant.

Dans un mode de réalisation particulier, le module de communication de l'équipement centralisé et le deuxième module de communication de l'équipement distant mettent en oeuvre un mécanisme de cryptage/décryptage de leurs communications.

En variante, à la place du protocole HTTP, les équipements peuvent communiquer selon le protocole HTTPS pour réaliser intrinsèquement cette fonction de cryptage/décryptage.

Cette caractéristique permet avantageusement d'assurer la confidentialité des données biologiques échangées sur le réseau.

Dans un mode particulier de réalisation, l'équipement distant comporte une unité de traçabilité du tube et/ou d'un conditionnement comportant les prélèvements biologiques à analyser de manière à réconcilier le flux physique du tube (ou conditionnement) et du flux de données échangées entre les laboratoires.

Dans un mode particulier de réalisation, l'équipement centralisé comporte des moyens d'accès à une base de données des analyses pouvant être réalisées par les automates du laboratoire sous-traitant, l'équipement distant étant apte à télécharger le contenu de cette base de données d'examens dans une mémoire accessible par au moins un terminal du système d'information du laboratoire commanditaire.

En pratique, le laboratoire sous-traitant peut mettre à jour cette base de données (ou catalogue) de façon régulière permettant ainsi la mise à jour des codes d'examens et des codes de résultats par les laboratoires commanditaires. Cette base de données peut par exemple contenir des fichiers échangeables au format HL7.

Dans un mode de réalisation, l'équipement distant télécharge les mises à jour de ces fichiers dans sa mémoire, lorsqu'il se connecte à l'équipement centralisé, par exemple en utilisant un service Web. L'utilisateur du système d'informations du laboratoire de biologie commanditaire peut alors être prévenu de cette mise à jour, par exemple par courrier électronique.

Dans un mode de réalisation de l'invention, l'équipement distant comporte des moyens de mise en oeuvre d'une application permettant de visualiser le catalogue d'examens ainsi mis à disposition par le laboratoire de biologie sous-traitant.

Dans un mode particulier de réalisation, l'équipement distant comporte une unité de supervision des communications avec l'équipement centralisé.

Cette unité de supervision permet l'administration de l'équipement distant, par exemple via une interface Web et notamment :
- la supervision des demandes envoyées et non encore traitées ;
- la supervision des demandes en cours d'envoi ;
- la supervision des demandes en cours de réception;
- la supervision des demandes envoyées/reçues ;
- la recherche d'une demande par numéro de conditionnement, numéro de patient, numéro de dossier, ...
- la supervision des versions du catalogue des examens pouvant être réalisés par le laboratoire sous-traitant, éventuellement avec le différentiel.

Dans un mode de réalisation, le ou les automates d'analyses d'échantillons du laboratoire sous-traitant sont connectés à un concentrateur relié à un système d'informations apte à :
- recevoir ladite demande d'analyse au format normalisé de présentation ;
- transmettre cette demande d'analyse à un automate d'analyses d'échantillons ;
- générer le résultat d'analyse ; et
- transmettre ce résultat d'analyse à l'équipement distant via le module de communication du laboratoire sous-traitant.

L'invention vise aussi l'équipement central et l'équipement distant tel que mentionnés ci-dessus.

Ainsi et plus précisément, l'invention concerne un équipement destiné à être installé dans un laboratoire de biologie commanditaire, cet équipement comportant :
- un premier module de communication connecté à un concentrateur d'automates d'analyses d'échantillons et apte à recevoir via ce concentrateur et conformément à un protocole standardisé interprétable par le concentrateur, une demande d'analyse;
- un deuxième module de communication apte à envoyer à un module de communication distant, via un réseau de communication, cette demande d'analyse et à recevoir en réponse un résultat d'analyse ;
- le premier module de communication étant apte à envoyer le résultat d'analyse au système d'information via ledit concentrateur selon ledit protocole standardisé.

L'invention vise aussi un équipement destiné à être installé dans un laboratoire de biologie sous-traitant, cet équipement comportant un module de communication apte à :
- recevoir, via un réseau de communication, une demande d'analyse et à transmettre cette demande selon un format normalisé de présentation de données biologiques à un système d'information relié via un concentrateur d'automates, à un automate apte à réaliser au moins une analyse correspondant à cette demande ;
- recevoir du système d'informations un résultat de cette analyse, et à envoyer ce résultat via le réseau de communication.

Comme mentionné précédemment, le système comporte un module de conversion. Ce module peut par exemple être incorporé dans l'équipement centralisé hébergé dans le laboratoire sous-traitant ou dans l'équipement distant hébergé dans le laboratoire commanditaire.

Par conséquent, l'invention vise également un équipement distant et un équipement centralisé comportant un module de conversion apte à :
- convertir une demande d'analyse selon un format normalisé de présentation de données biologiques pour traitement par un système d'information du laboratoire de biologie sous-traitant ; et à
- convertir un résultat d'analyse conformément au protocole standardisé pour que ce résultat puisse être communiqué au système d'information du laboratoire commanditaire via le concentrateur de ce laboratoire.

### Brève description des dessins

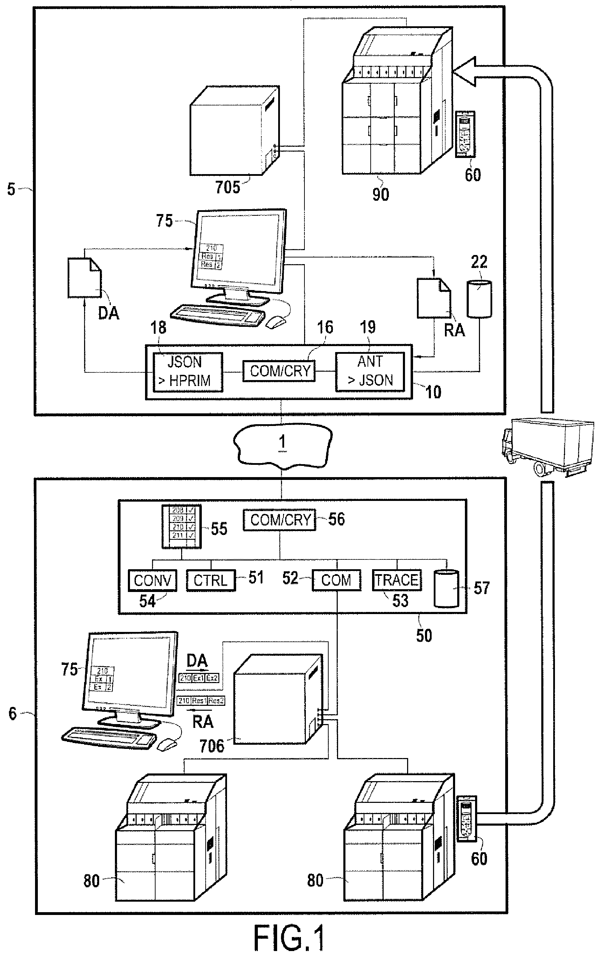

D'autres caractéristiques et avantages de la présente invention ressortiront de la description faite ci-dessous, en référence à la Figure 1 annexée qui illustre un système d'analyses d'échantillons conforme à l'invention et dépourvu de tout caractère limitatif.

### Description détaillée d'un mode de réalisation

La **Figure 1** représente un système pour l'analyse d'échantillons conforme à l'invention.

Dans cet exemple, on se place dans le contexte où l'invention est utilisée par des laboratoires d'analyses médicales.

Dans cet exemple on considère un laboratoire d'analyses médicales commanditaire **6** et un laboratoire d'analyses médicales sous-traitant **5.**

Conformément à l'invention, le laboratoire d'analyses médicales commanditaire **6** et le laboratoire d'analyses médicales sous-traitant **5** sont interconnectés via un réseau de communication **1,** le laboratoire sous-traitant **5** comportant un équipement **10** (« équipement centralisé au sens de l'invention ») interconnecté à un équipement **50** installé dans le laboratoire commanditaire **6** (« équipement distant au sens de l'invention »).

On supposera, dans cet exemple, qu'un patient se rend au laboratoire d'analyses médicales commanditaire **6** pour passer des examens médicaux.

A son arrivée au laboratoire, une assistante accueille le patient et enregistre ses informations personnelles, par exemple son nom, son prénom et son numéro de sécurité sociale dans un terminal relié au système d'informations **75** du laboratoire commanditaire **6.**

Puis, différents prélèvements sont réalisés, par exemple un examen d'urine et une prise de sang. Comme de façon classique, le sang du patient est conservé dans un tube à essai primaire. On suppose dans cet exemple que cinq examens ou analyses doivent être réalisés sur ce prélèvement.

Parmi ces cinq examens quatre peuvent être réalisés par les automates d'analyses d'échantillons **80** du laboratoire d'analyses médicales commanditaire **6,** mais pas le cinquième.

De façon connue, les automates **80** du laboratoire d'analyses médicales commanditaire **6** sont interconnectés à un concentrateur d'automates **706** auquel est également relié le terminal précédemment utilisé pour enregistrer les informations personnelles du patient.

Dans cet exemple, les automates **80** communiquent avec le concentrateur **706** conformément au protocole standardisé ASTM (American Society for Testing and Materials).

Un tube secondaire est généré par un automate **80** à partir du tube primaire pour contenir l'échantillon sanguin sur lequel doit être réalisé le cinquième examen.

Dans le mode de réalisation décrit ici, le personnel du laboratoire d'analyses médicales commanditaire **6** peut accéder via un terminal à une mémoire **57** de l'équipement distant **50,** dans laquelle sont mémorisés les examens pouvant être réalisés par les automates **90** du laboratoire d'analyses médicales sous-traitant **5.** Nous supposerons que le cinquième examen devant être réalisé dans cet exemple peut effectivement être réalisé par un automate **90** du laboratoire sous-traitant **5.**

Dans l'exemple de réalisation décrit ici, le personnel du laboratoire commanditaire **6** peut en outre accéder à une base de données **55** de l'équipement distant **50** dans laquelle sont mémorisés des numéros (**208** à **211**) réservés au laboratoire d'analyses médicales sous-traitant **5.** Nous supposerons dans cet exemple que le personnel sélectionne le numéro **210.**

Le personnel imprime alors une étiquette sur laquelle apparaissent ce numéro **210** et l'identité du patient, colle cette étiquette sur le tube secondaire et place le tube secondaire dans un sachet **60.** Dans l'exemple de réalisation décrit ici, le sachet **60** comportant le tube secondaire ainsi étiqueté est envoyé par camion à destination du laboratoire d'analyses médicales sous-traitant **5.**

Dans le mode de réalisation décrit ici, l'équipement distant **50** comporte un module **53** permettant de réaliser la traçabilité des demandes d'analyse sous-traitées en association avec les tubes secondaires physiques envoyés au laboratoire sous-traitant pour analyse.

Ce module de traçabilité permet avantageusement au personnel du laboratoire commanditaire de connaître à tout moment la localisation des tubes secondaires dès qu'ils quittent le laboratoire, par exemple au moyen d'un code-barres imprimé sur l'étiquette et scanné par chacun des intervenants dans la chaîne de transport du conditionnement de ces tubes.

Conformément à l'invention, l'équipement distant **50** du laboratoire commanditaire **6** est interconnecté au concentrateur d'automates **706** et communique avec ce concentrateur d'automates **706** conformément au protocole standardisé ASTM.

Cette caractéristique est particulièrement avantageuse car elle permet au personnel du laboratoire commanditaire **6** de communiquer avec le laboratoire sous-traitant **5** exactement de la même façon qu'elle communique avec les automates **80** locaux. Autrement dit, l'installation d'un équipement distant **50** dans le laboratoire commanditaire **6** ne nécessite aucune modification de l'infrastructure informatique ou logicielle de ce laboratoire puisqu'il suffit de connecter l'équipement distant **50** sur un port du concentrateur **706** normalement prévu pour connecter un automate **80.**

Cet équipement distant **50** comporte en particulier :
- un premier module de communications **52** pour communiquer, conformément au protocole ASTM avec le concentrateur d'automates **706 ;**
- et un deuxième module de communication **56** pour communiquer, via le réseau de télécommunications **1** avec l'équipement centralisé **10** installé dans le laboratoire d'analyses médicales sous-traitant **5.**

Comme décrit précédemment dans l'exemple décrit ici, l'équipement distant **50** comporte en outre une mémoire **57** contenant le catalogue des examens pouvant être réalisés par le laboratoire sous-traitant **5** et une base de données **55** des numéros réservés pour ce laboratoire. En outre, dans cet exemple, l'équipement distant **50** comporte un module **51** pour superviser les communications en cours et/ou passées entre les laboratoires.

Conformément à l'invention, le système comporte, et ce de façon très avantageuse, un module de conversion **54** apte à convertir une demande d'analyse **DA** reçue selon le protocole standardisé (par exemple ASTM) selon un format de présentation normalisé, par exemple HPRIM (Harmonie et promotion de l'informatique médicale), de sorte que cette demande d'analyse peut être interprétée directement par le système d'information **75** du laboratoire d'analyses médicales sous-traitant **5.**

Dans le mode de réalisation décrit ici, ce module de conversion est intégré à l'équipement distant **50** du laboratoire commanditaire **6,** mais en variante il pourrait être intégré à l'équipement centralisé **10** du laboratoire sous-traitant **5.**

La demande d'analyse **DA** convertie au format HPRIM est envoyée par l'équipement distant **50,** via le réseau de télécommunications **1,** à l'équipement centralisé **10** du laboratoire sous-traitant **5.** A cet effet, l'équipement centralisé **10** comporte des moyens de communication **16** compatibles avec les deuxièmes moyens de communications **56** de l'équipement distant **50.**

Dans le mode de réalisation décrit ici, ces modules de communications **16, 56** communiquent de façon cryptée. A cet effet, chacun de ces modules comporte des moyens connus de cryptage et de décryptage des communications.

Dans le mode de réalisation décrit ici, le module de communications **56** du laboratoire d'analyses médicales commanditaire **6** encapsule la demande d'analyse **DA** au format HPRIM dans un web service par exemple conforme au protocole JSON ou REST.

L'équipement centralisé **10** comporte par conséquent un module **18** apte à décapsuler la demande d'analyse **DA** reçue du laboratoire commanditaire **6** pour restituer la demande **DA** au format HPRIM directement interprétable par le système d'informations **75** du laboratoire sous-traitant **5.**

Nous supposerons que le tube secondaire dans son conditionnement **60** est effectivement livré au laboratoire sous-traitant **5,** que celui-ci est placé dans un automate **90** de ce laboratoire pour réaliser le cinquième examen devant être opéré sur le sang précédemment prélevé dans le laboratoire commanditaire **6** et que le personnel du laboratoire sous-traitant **5** pilote cet automate, via un concentrateur d'automates **705** pour réaliser cet examen.

Le résultat de l'analyse **RA** comporte dans cet exemple un premier résultat **RES1** et un deuxième résultat **RES2** accessibles par le personnel du laboratoire sous-traitant **5** via le système d'information **75.** Ce personnel produit un résultat d'analyse **RA.**

Conformément à l'invention, le résultat d'analyse **RA** est envoyé au laboratoire commanditaire **6** via le réseau de communications **1.**

Dans cet exemple, les laboratoires communiquent entre eux en utilisant un web service conforme au protocole JSON ; le résultat d'analyse **RA** est donc converti par un module **19** au format JSON au sein de l'équipement centralisé **10.**

Conformément à l'invention, le module de conversion **54** convertit le résultat d'analyse **RA** conformément au protocole standardisé interprétable par les concentrateurs d'automates **706,** par exemple ASTM, le résultat pouvant ainsi être envoyé par le premier module de communications **52,** au système d'informations **75** du laboratoire commanditaire **6** via le concentrateur **706.**

Ainsi, et de façon très avantageuse, le personnel du laboratoire commanditaire **6** obtient les résultats exactement de la même façon que si l'analyse avait été réalisée par un automate **80** local à ce laboratoire

L'invention n'est pas limitée aux exemples décrits et représentés car diverses modifications peuvent y être apportées sans sortir de son cadre.

## Revendications

1. Système pour l'analyse d'échantillons comportant :
- un équipement centralisé **(10)** relié à un système d'information **(75)** d'un laboratoire de biologie sous-traitant **(5)** opérant au moins un automate d'analyses d'échantillons **(90) ;** et
- au moins un équipement distant **(50)** destiné à être installé dans un laboratoire de biologie commanditaire **(6),** ledit équipement distant **(50)** étant relié à l'équipement centralisé **(10)** par un réseau de communication **(1),** ledit équipement distant **(50)** comportant :
- un premier module de communication **(52)** connecté à un concentrateur **(706)** d'automates d'analyses d'échantillons **(80)** et apte à recevoir d'un système d'information **(75)** du laboratoire commanditaire, via ledit concentrateur **(706)** et conformément à un protocole standardisé **(STD)** interprétable par le concentrateur, une demande d'analyse **(DA)** pouvant être réalisée par un automate **(90)** dudit laboratoire sous-traitant **(5);** et
- un deuxième module de communication **(56)** apte à envoyer ladite demande d'analyse **(DA)** à un module de communication **(16)** dudit équipement centralisé **(10)** et à recevoir en réponse un résultat d'analyse **(RA);**
- ledit premier module de communication **(52)** étant apte à envoyer ledit résultat d'analyse **(RA)** audit système d'information **(75)** du laboratoire commanditaire **(6)** via ledit concentrateur **(706)** selon ledit protocole standardisé ;
- ledit système comportant un module de conversion **(54)** apte à :
- convertir ladite demande d'analyse **(DA)** selon un format normalisé de présentation de données biologiques **(PRES)** pour traitement par un système d'information dudit laboratoire de biologie sous-traitant ; et à
- convertir ledit résultat d'analyse **(RA)** conformément audit protocole standardisé **(STD)** pour que le résultat puisse être communiqué au système d'information **(75)** du laboratoire commanditaire **(6)** via ledit concentrateur **(706)** de ce laboratoire.

2. Système **(100)** selon la revendication 1 **caractérisé en ce que** le module de communication **(16)** de l'équipement centralisé **(10)** et le deuxième module de communication **(56)** de l'équipement distant **(50)** encapsulent respectivement ladite demande d'analyse **(DA)** et ledit résultat d'analyse **(RA)** dans un Service Web, par exemple conforme au protocole JSON et/ou REST.

3. Système **(100)** selon la revendication 1 ou 2 **caractérisé en ce que** le module de communication **(16)** de l'équipement centralisé **(10)** et le deuxième module de communication **(56)** de l'équipement distant (50) mettent en oeuvre un mécanisme de cryptage/décryptage de leurs communications.

4. Système **(100)** selon l'une quelconque des revendications 1 à 3 **caractérisé en ce que** l'équipement distant **(50)** comporte une unité **(51)** de supervision des communications avec l'équipement centralisé **(10).**

5. Système **(100)** selon l'une quelconque des revendications 1 à 4, **caractérisé en ce que** l'équipement distant **(50)** comporte une unité **(53)** de traçabilité d'au moins un tube et/ou d'un conditionnement **(60)** comportant les prélèvements biologiques sur lesquels doivent être réalisés ladite demande d'analyse **(DA).**

6. Système **(100)** selon l'une quelconque des revendications 1 à 5, dans lequel ledit équipement centralisé **(10)** comporte des moyens d'accès à une base de données **(22)** des analyses pouvant être réalisées par ledit au moins un automate **(90)** dudit laboratoire sous-traitant **(5),** ledit équipement distant (50) étant apte à télécharger le contenu de ladite base de données d'examens **(22)** dans une mémoire **(57)** accessible par au moins terminal du système d'information **(75)** du laboratoire commanditaire **(6).**

7. Système **(100)** selon l'une quelconque des revendications 1 à 6, **caractérisé en ce que** ledit au moins un automate d'analyses d'échantillons **(90)** dudit laboratoire sous-traitant **(5)** est connecté à un concentrateur **(705)** relié à un système d'informations **(75)** apte à :
- recevoir ladite demande d'analyse **(DA)** au format normalisé de présentation **(PRES);**
- transmettre ladite demande d'analyse **(DA)** audit automate d'analyses d'échantillons **(90) ;**
- générer ledit résultat d'analyse **(RA) ;** et
- transmettre ledit résultat d'analyse **(RA)** audit équipement distant **(50)** via ledit module de communication **(16)** du laboratoire sous-traitant **(5).**

8. Équipement **(50)** destiné à être installé dans un laboratoire de biologie commanditaire, ledit équipement comportant :
- un premier module de communication **(52)** connecté à un concentrateur **(706)** d'automates d'analyses d'échantillons **(80)** et apte à recevoir via ledit concentrateur **(706)** et conformément à un protocole standardisé **(STD)** interprétable par ledit concentrateur **(706),** une demande d'analyse **(DA);**
- un deuxième module de communication **(56)** apte à envoyer à un module de communication distant **(16),** via un réseau de communication **(1)** distant, ladite demande d'analyse **(DA)** et à recevoir en réponse un résultat d'analyse **(RA);**
- ledit premier module de communication **(52)** étant apte à envoyer ledit résultat d'analyse **(RA)** audit système d'information **(75)** via ledit concentrateur **(706)** selon ledit protocole standardisé **(STD).**

9. Équipement **(10)** destiné à être installé dans un laboratoire d'analyses de biologie sous-traitant, ledit équipement comportant un module de communication **(16)** apte à :
- recevoir, via un réseau de communication **(1)** une demande d'analyse **(DA)** et à transmettre ladite demande **(DA)** selon un format normalisé de présentation de données biologiques **(PRES)** à un système d'information **(75)** relié via un concentrateur d'automates **(705)** à un automate **(90)** apte à réaliser au moins une analyse correspondant à ladite demande **(DA) ;**
- recevoir dudit système d'informations **(75)** un résultat **(RA)** de ladite au moins une analyse et à envoyer ledit résultat via ledit réseau de communication **(1).**

10. Equipement selon la revendication 8 ou 9 **caractérisé en ce qu'**il comporte un module de conversion **(54)** apte à :
- convertir ladite demande d'analyse **(DA)** selon un format normalisé de présentation de données biologiques **(PRES)** pour traitement par un système d'information dudit laboratoire de biologie sous-traitant ; et à
- convertir ledit résultat d'analyse **(RA)** conformément audit protocole standardisé **(STD)** pour que ce résultat puisse être communiqué au système d'information **(75)** du laboratoire commanditaire **(6)** via ledit concentrateur **(706)** de ce laboratoire.
